# FASCICULE DE BREVET EUROPEEN

(11) **EP 0 888 363 B1**
(45) Date de publication et mention de la délivrance du brevet: **17.10.2001**
(21) Numéro de dépôt: 97914407.8
(22) Date de dépôt: 14.03.1997
(51) Int. Cl.: C07F 7/08, A61K 7/13

(54) **COLORANTS SILOXANIQUES, COMPOSITIONS LES CONTENANT ET UTILISATIONS**
SILOXANFARBSTOFFE, ZUSAMMENSETZUNGEN, DIE SIE ENTHALTEN UND IHRE VERWENDUNG
SILOXANE DYES, COMPOSITIONS CONTAINING SAME AND USES THEREOF

(30) Priorité: 18.03.1996 FR 9603345
(43) Date de publication de la demande: 07.01.1999
(73) Titulaire: L'OREAL, 75008 Paris (FR)
(72) Inventeur: RICHARD, Hervé, F-93420 Villepinte (FR); LEDUC, Madeleine, Rue des Boulets F-75011 Paris (FR); LAGRANGE, Alain, F-77450 Coupvray (FR)
(74) Mandataire: Miszputen, Laurent
(86) Numéro de dépôt international: FR9700468
(87) Numéro de publication internationale: WO9734904

(56) Documents cités:
- EP-A- 0 287 479
- FR-A- 2 642 968
- GB-A- 2 018 797
- US-A- 4 892 918
- CHEMICAL ABSTRACTS, vol. 88, no. 23, 5 Juin 1978 Columbus, Ohio, US; abstract no. 170282c, XP002019747 & JP 78 005 124 A (SHIN-ETSU CHEMICAL INDUSTRY CO., LTD.) 18 Janvier 1978

## Description

L'invention concerne des composés de type diorganosiloxane à fonction nitroaniline constituant une nouvelle famille de colorants utilisables dans le domaine de la cosmétique.

On a déjà décrit et proposé des colorants à base de silicium pour colorer des fibres organiques naturelles et synthétiques ou des matières minérales. De tels colorants comme ceux décrits dans les brevets belges N° 875 160 - 875 230 ou encore américains N° 2 925 313 - 2 963 338 sont néanmoins réactifs en raison des groupes hydrolysables qui subsistent sur l'atome de silicium, ce qui peut générer une certaine instabilité ou évolution indésirable dans certaines conditions d'utilisation. A cet inconvénient, vient s'ajouter un poids moléculaire parfois difficilement contrôlable.

On connaît, dans l'état de la technique, des colorants polysiloxaniques, comportant dans leur chaîne au moins deux groupements aromatiques chromophores de type azoïque ou anthrone.
De tels colorants sont décrits dans le brevet américain N° 4 381 260.

Dans le domaine de la cosmétique, et notamment, par exemple, dans la coloration capillaire, ou dans la fabrication des fards pour les lèvres, le visage, les cils ou les sourcils, on recherche des colorants directs, c'est-à-dire des colorants qui modifient la nuance naturelle de façon temporaire, qui présentent une bonne innocuité, et qui soient stables, en particulier à la lumière, aux lavages et aux intempéries.

La présente invention vise à obtenir lesdits avantages, en proposant de nouveaux composés du type diorganosiloxane, linéaires ou cycliques, à fonction nitroaniline, qui sont chimiquement et physiquement stables, et qui présentent une très bonne affinité pour les fibres, notamment pour les fibres kératiniques humaines telles que les cheveux.
Ces nouveaux composés présentent en outre une bonne innocuité, les rendant particulièrement appropriés à une utilisation comme colorants dans des, ou pour la préparation de, compositions cosmétiques destinées à la teinture des fibres kératiniques humaines et notamment des cheveux ou au maquillage.

La présente invention a ainsi pour objet des composés qui sont caractérisés par le fait qu'ils répondent à l'une des formules (1) ou (2) suivantes : 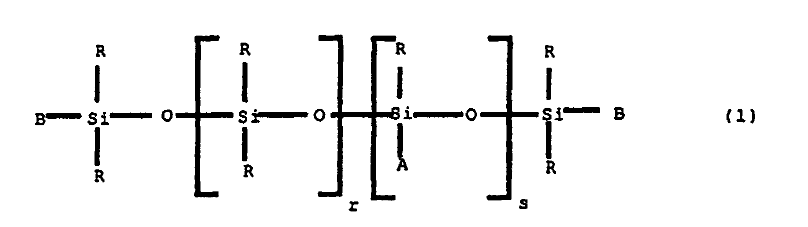 ou 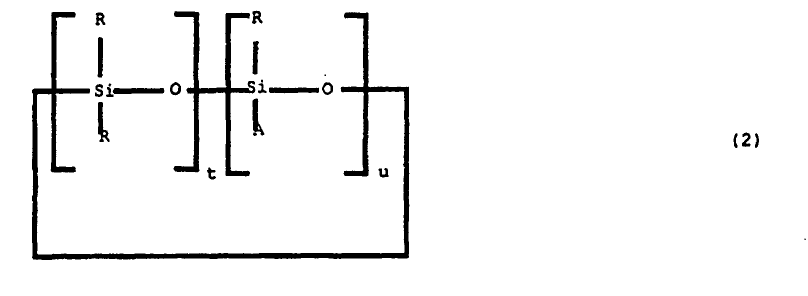 formules (1) ou (2) dans lesquelles :
- R, identiques ou différents, sont choisis parmi les radicaux alkyles en C₁-C₁₀, phényle et trifluoro-3,3,3 propyle, au moins 80% en nombre des radicaux R étant méthyle,
- B, identiques ou différents, sont choisis parmi les radicaux R ci-dessus et le radical A défini ci-après,
- r est un nombre entier compris entre 0 et 50 inclusivement, et s est un nombre entier compris entre 0 et 20 inclusivement, avec la condition que si s est nul alors au moins l'un des deux symboles B désigne A,
- u est un nombre entier compris entre 1 et 6 inclusivement, et t est un nombre entier compris entre 0 et 10 inclusivement, étant entendu que t + u est égal ou supérieur à 3,
- et le symbole A désigne un radical monovalent lié directement à un atome de silicium, et qui répond à la formule (3) suivante: 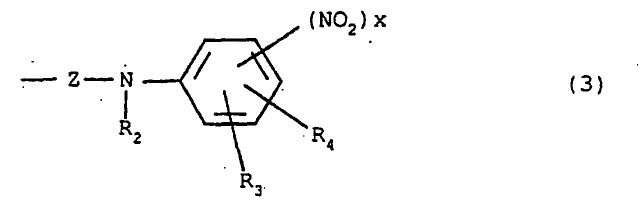 formule (3) dans laquelle :

- Z est le radical divalent : 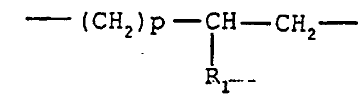
- x est 1 ou 2,
- p représente un nombre entier compris entre 0 et 10, inclusivement,
- R₁ représente l'hydrogène ou un radical alkyle en C₁-C₄,
- R₂ représente l'hydrogène ou un radical alkyle en C₁-C₄, ou le radical divalent Z défini ci-dessus,
- R₃ représente l'hydrogène ou un radical NR₅R₆ dans lequel R₅ et R₆ représentent l'hydrogène ou un radical alkyle en C₁-C₄, un radical mono- ou di-hydroxyalkyle en C₂-C₄ ou le radical divalent Z,
- R₄ représente l'hydrogène, un radical OH ou halogène, un radical alkyle en C₁-C₄, ou un radical alkoxy en C₁-C₄.

Dans les formules (1) ou (2) ci-dessus, A représente donc le groupement nitroaniline qui, après fixation sur la chaîne siliconée de départ, confère aux composés de type diorganosiloxane linéaire [formule (1)] ou cyclique [formule (2)1 des propriétés colorantes. Les radicaux alkyle peuvent être linéaires ou ramifiés et choisis notamment au sein des radicaux méthyle, éthyle, n-propyle, isopropyle, n-butyle, isobutyle, ter.-butyle, n-amyle, isoamyle, néopentyle, n-hexyle, n-heptyle, n-octyle, éthyl-2 hexyle et ter.-octyle. Les radicaux alkyle R, et B préférés selon l'invention sont les radicaux méthyle, éthyle, propyle, n-butyle, n-octyle et éthyl-2 hexyle. Encore plus préférentiellement, les radicaux R, et B sont tous des radicaux méthyle.

Parmi les diorganosiloxanes linéaires ou cycliques rentrant dans le cadre de la présente invention, on préfère plus particulièrement les dérivés statistiques ou bien définis à blocs présentant au moins l'une, et encore plus préférentiellement l'ensemble, des caractéristiques suivantes :
- R est alkyle et encore plus préférentiellement est méthyle,
- B est alkyle et encore plus préférentiellement méthyle [cas des composés linéaires de formule (1)],
- r est compris entre 0 et 3 inclusivement ; s est compris entre 0 et 3 inclusivement (cas des composés linéaires de formule (1)),
- t+u est compris entre 3 et 5 [cas des composés cycliques de formule (2)],
- R₁ est hydrogène ou méthyle,
- p est égal à 1,
- x est 1 ou 2,
- R₂ et R₄ désignent hydrogène,
- R₃ est hydrogène ou un radical NR₅R₆ dans lequel R₅ et R₆ représentent l'hydrogène ou un radical mono- ou di-hydroxyalkyle en C₂-C₄,
et plus avantageusement encore, l'ensemble des caractéristiques suivantes :
- R est alkyle et encore plus préférentiellement est méthyle,
- B est alkyle et encore plus préférentiellement méthyle [cas des composés linéaires de formule (1)],
- Z est le radical divalent : 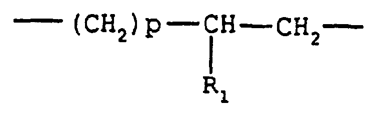
- R₁ est hydrogène ou méthyle,
- p est égal à 1,
- r est nul, et s est égal à 1 (cas des composés linéaires de formule (1)),
- u=1 et t=2 [cas des composés cycliques de formule (2)].
- x est 1 ou 2,
- R₂ et R₄ désignent hydrogène,
- R₃ est hydrogène ou un radical NR₅R₆ dans lequel R₅ et R₆ représentent l'hydrogène ou un radical mono- ou di-hydroxyalkyle en C₂-C₄.

Selon l'invention, les composés plus particulièrement préférés sont les suivants :

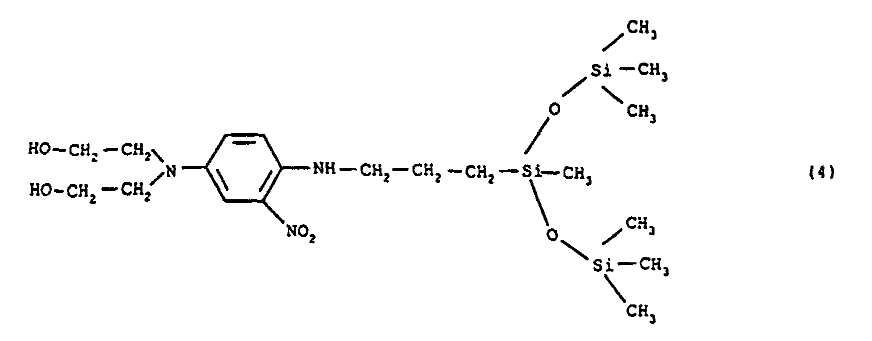

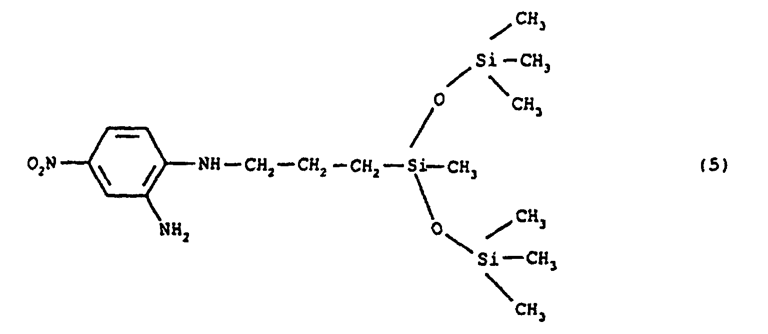

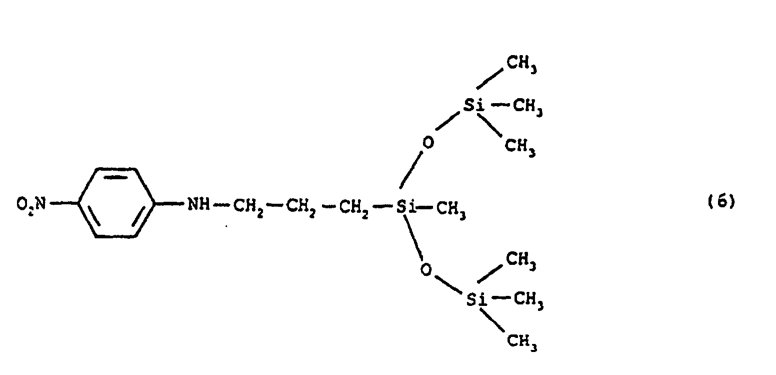

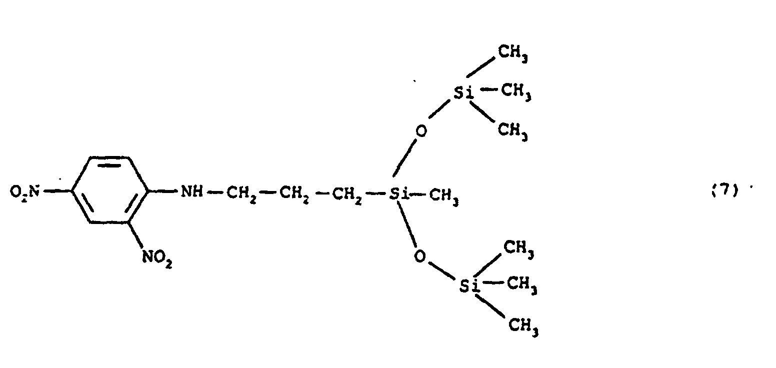

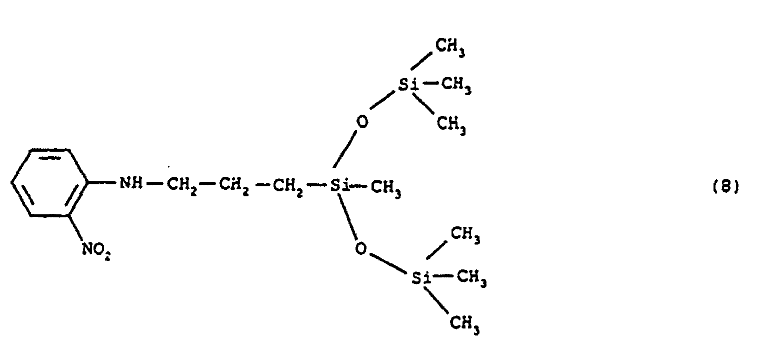

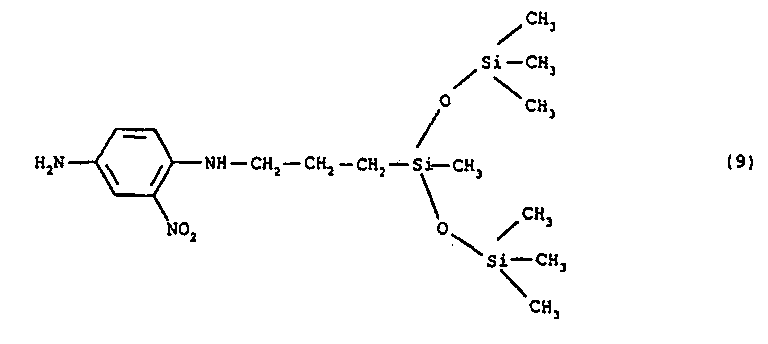

Pour préparer les colorants siloxaniques de formules (1) et (2), on peut procéder classiquement (voie 1) en mettant en oeuvre une réaction d'hydrosilylation à savoir, 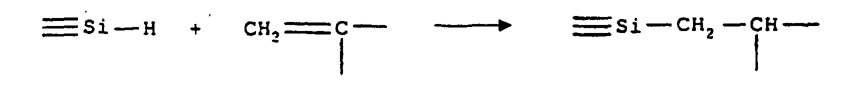 à partir de la silicone correspondante, dans laquelle, par exemple, tous les radicaux A sont des atomes d'hydrogène. Cette silicone de départ est dénommée par la suite dérivé à SiH ; les groupes SiH peuvent être présents dans la chaîne et/ou aux extrémités de la chaîne silicone. Ces dérivés à SiH sont des produits bien connus dans l'industrie des silicones et sont généralement disponibles dans le commerce. Ils sont par exemple décrits dans les brevets américains US-A-3 220 972, US-A- 3 697 473 et US-A- 4.340 709.

Ce dérivé à SiH peut être donc représenté, soit par la formule (1bis) suivante : 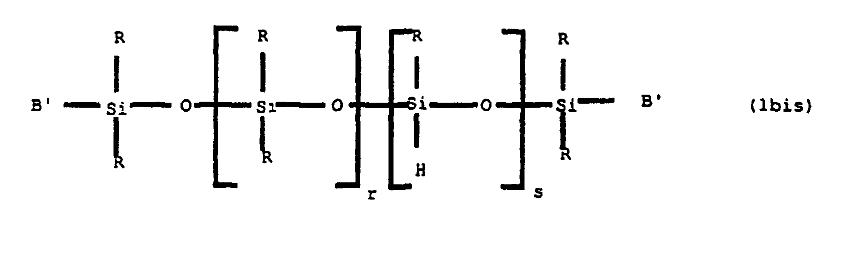 dans laquelle R, r et s ont la signification donnée ci-dessus pour la formule (1) et les radicaux B', identiques ou différents, sont choisis parmi les radicaux R et un atome d'hydrogène,
soit par la formule (2bis) suivante : 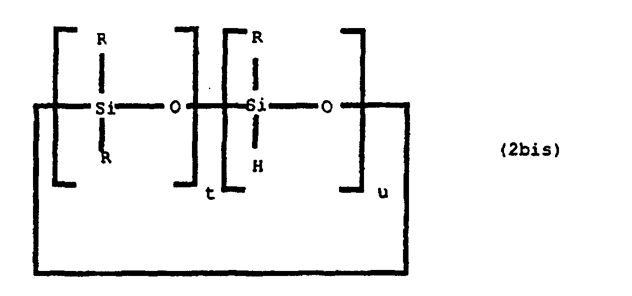 dans laquelle R, t et u ont la signification donnée ci-dessus pour la formule (2).

Sur ce dérivé à SiH de formule (1bis) ou (2bis), on effectue donc une réaction d'hydrosilylation classique, opérée en présence d'une quantité catalytiquement efficace d'un catalyseur au platine, sur une nitroaniline de formule (3bis) suivante : 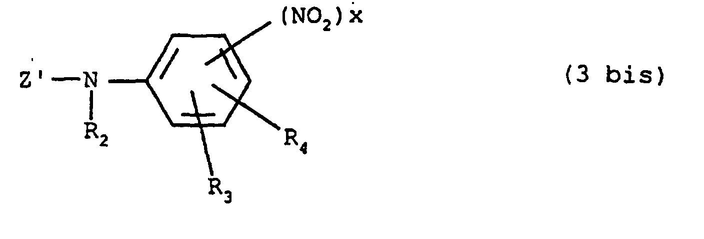 dans laquelle,
- Z' est le radical : 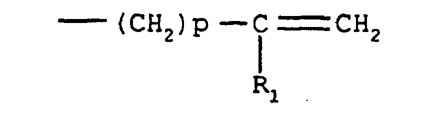
- R₂ est hydrogène, alkyle en C₁-C₄ ou le radical Z',
- R₃ est hydrogène, un radical NR₅R₆ dans lequel R₅ et R₆ représentent l'hydrogène ou un radical alkyle en C₁-C₄, un radical mono- ou di-hydroxyalkyle en C₂-C₄ ou le radical Z',
- x, p, R₁, et R₄ ont la signification donnée ci-dessus pour la formule (3),

Des procédés convenant à la préparation des produits de formule (3bis) ci-dessus sont notamment décrits dans la demande de brevet DE-42 40 684.

Une autre voie de synthèse possible (voie 2) convenant à la préparation des colorants polysiloxaniques de formules (1) et (2) consiste à partir des dérivés correspondant respectivement à la formule (1) ou à la formule (2) dans laquelle tous les radicaux A sont remplacés par le radical de formule (10) suivante : 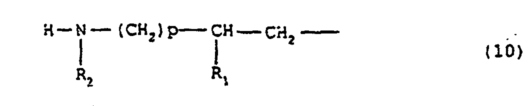 dans laquelle R₁, R₂ et p ont la même signification que dans la formule (3).

Les radicaux de formule (10) peuvent être présents dans la chaîne et/ou aux extrémités de la chaîne siliconée. Ces dérivés amino siloxanes de départ peuvent ainsi être représentés soit par la formule (1ter) suivante (dérivé amino siloxane linéaire) : 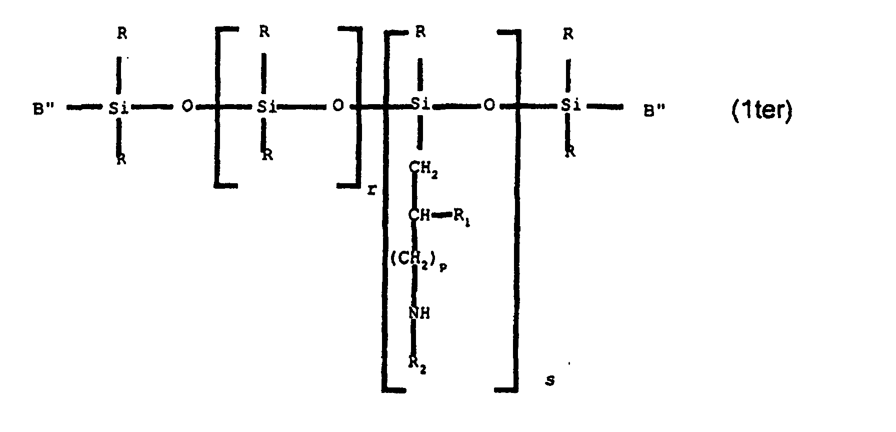 dans laquelle R, r et s ont la signification donnée ci-dessus pour la formule (1) et les radicaux B", identiques ou différents, sont choisis parmi les radicaux R et le radical de formule (10),
soit par la formule (2ter) suivante (dérivé amino siloxane cyclique) : 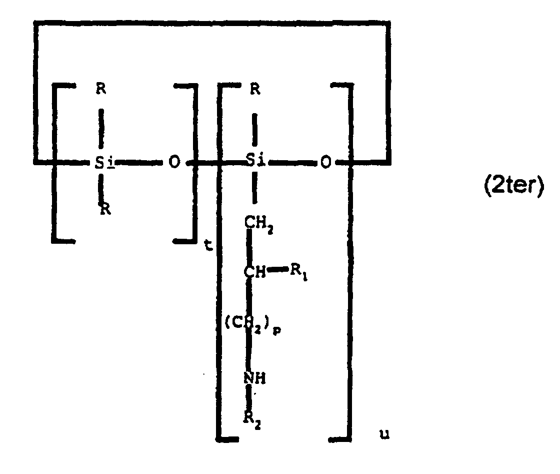 dans laquelle R, t et u ont la signification donnée ci-dessus pour la formules (2).

Les dérivés amino siloxanes de formule (1ter) ou (2ter) ci-dessus sont des produits bien connus dans l'industrie des silicones et sont généralement disponibles dans le commerce. Ils sont en outre notamment décrits dans la demande de brevet DE-A 3 702 631.

Sur ces dérivés amino siloxanes, on fait alors réagir le dérivé halogéné de formule (11) suivante: 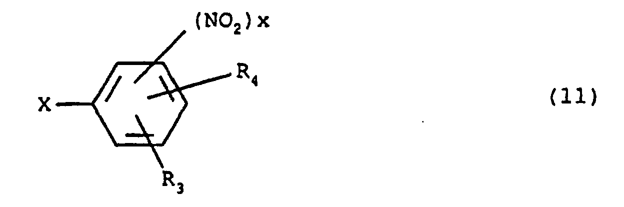 X étant plus particulièrement le fluor ou le chlore, et encore plus particulièrement le fluor.

La présente invention a aussi pour objet l'utilisation des composés de formule (1) ou (2) à titre de colorants dans une composition cosmétique, pharmaceutique ou alimentaire ou dans une composition tinctoriale destinée à la teinture des fibres naturelles ou synthétiques, ou des matières plastiques ou minérales.

L'invention a donc aussi pour objet une composition cosmétique, comprenant dans un milieu cosmétiquement acceptable, une quantité efficace d'au moins un composé de formule (1) ou (2) définie ci-avant.

Les composés de formule (1) ou (2) sont généralement présents dans des proportions comprises entre environ 0,01 et 10%, de préférence entre environ 0,1 et 5% en poids, par rapport au poids total de la composition cosmétique.

Lorsque la composition de l'invention est une composition cosmétique, elle peut être utilisée comme composition tinctoriale des fibres kératiniques et en particulier comme composition de teinture directe des cheveux, comme composition de teinture d'oxydation capillaire contenant au moins un colorant d'oxydation et le ou les composés de formule (1) ou (2) à titre de colorants directs. Elle peut également être utilisée comme composition de maquillage telle que les produits pour les lèvres ou le visage, les cils, les sourcils, bâton de rouge à lèvres, fard à paupière, fard à joues, fond de teint, ligneur encore appelé «eye-liner», ou mascara et vernis à ongles.

Le milieu cosmétiquement accceptable est alors de préférence un milieu constitué par de l'eau et/ou des solvants organiques acceptables sur le plan cosmétique, et plus particulièrement, des alcools, des glycols ou éthers de glycol, dans des concentrations comprises entre environ 0,5 et 20% et, de préférence, entre environ 2 et 10% en poids par rapport au poids total de la composition. Il peut également contenir des corps gras tels que des huiles et des cires.
Ladite composition cosmétique peut contenir en outre tout autre adjuvant utilisé habituellement en cosmétique, suivant l'application envisagée, et par exemple, des agents tensio-actifs bien connus de l'état de la technique et de type anionique, cationique, non-ionique, amphotère, zwittérionique ou leurs mélanges, des agents épaississants, des agents anti-oxydants, des parfums, des agents séquestrants, des agents dispersants, des agents de conditionnement, des agents conservateurs, des agents opacifiants, etc...
Bien entendu, l'homme de l'art veillera à choisir le ou les éventuels composés complémentaires mentionnés ci-avant, de manière telle que les propriétés avantageuses attachées intrinsèquement à la composition tinctoriale selon l'invention ne soient pas, ou substantiellement pas, altérées par la ou les adjonctions envisagées.
La composition cosmétique selon l'invention peut être formulée à pH acide, neutre ou alcalin, le pH pouvant varier par exemple de 4 à 11 et de préférence de 5 à 10, et pouvant être ajusté au moyen d'agents d'alcalinisation ou d'agents d'acidification antérieurement bien connus.
L'invention concerne également un procédé de teinture des fibres kératiniques humaines, et notamment des cheveux, par coloration directe, consistant à laisser agir une composition tinctoriale renfermant au moins un colorant de formule (1) ou (2) sur les fibres kératiniques sèches ou humides. On peut utiliser la composition selon l'invention en tant que composition non rincée, c'est-à-dire qu'après application de la composition sur les fibres, on sèche sans rinçage intermédiaire. Dans les autres modes d'application, on laisse agir la composition sur les fibres pendant un temps de pose variant entre 3 et 60 minutes environ, de préférence entre 5 et 45 minutes environ, on rince, éventuellement on lave et on rince à nouveau, puis on sèche.
Les exemples qui suivent illustrent l'invention sans toutefois en limiter la portée.

### EXEMPLE 1

Dans un ballon tout équipé, on introduit 5,62 g (0,02 mole) du composé de formule : 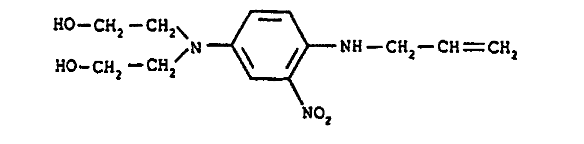 20 ml de toluène anhydre, et 100 µl de catalyseur au platine (complexe à 3-3,5% de Pt dans du cyclovinylméthylsiloxane, vendu par la société Hüls sous la marque Petrarch PC085). Le mélange est porté à 80°C sous azote, puis on ajoute goutte à goutte, 5,56 g (0,025 mole) d'heptaméthyltrisiloxane. Après trois heures d'agitation à 80°C sous azote, on concentre le milieu, on évapore le solvant et le siloxane en excès.
L'huile obtenue est chromatographiée sur silice (éluant : Heptane /Acétate d'éthyle/50:50). On récupère ainsi 10,1 g du colorant siloxanique de formule (4) : 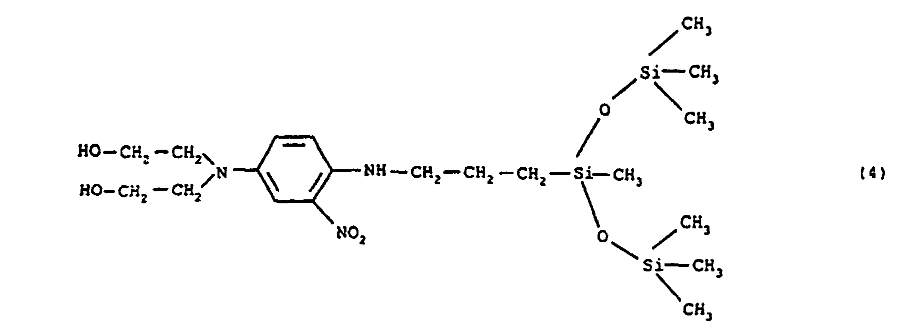 sous forme d'une huile épaisse violet foncé :

| - Analyse élémentaire pour **C**₂₀ **H**₄₁ **N**₃ **O**₆ **Si**₃ | | | | |
|---|---|---|---|---|
| théorie | **C** 47.68 | **H** 8.20 | **N** 8.34 | **Si** 16.72 |
| trouvé | **C** 47.75 | **H** 8.18 | **N** 8.19 | **Si** 16.54 |

### EXEMPLE 2

Dans un ballon tout équipé, on introduit 9,66 g (0,05 mole) du composé de formule : 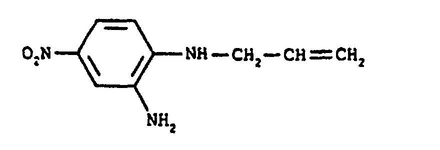 50 ml de toluène anhydre, et 100 µl de catalyseur au platine (complexe à 3-3,5% de Pt dans du cyclovinylméthylsiloxane, vendu par la société Hüls sous la marque Petrarch PC085). Le mélange est porté à 75°C sous azote, puis on ajoute goutte à goutte, 13,35 g (0,06 mole) d'heptaméthyltrisiloxane. Après six heures d'agitation à 80°C sous azote, on concentre le milieu, on évapore le solvant et le siloxane en excès.

L'huile obtenue est chromatographiée sur silice (éluant : Heptane/CH₂Cl₂/50:50). On récupère ainsi 9,9 g du colorant siloxanique de formule (5) : 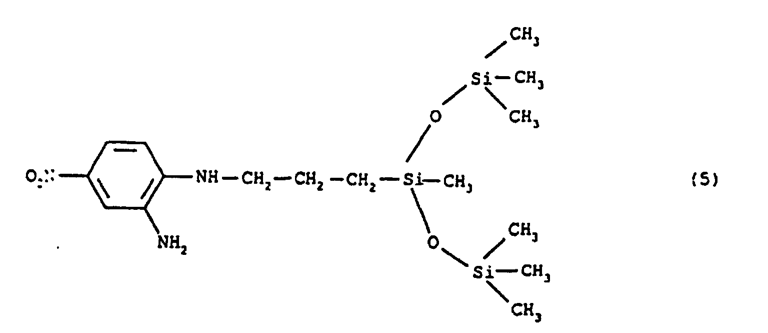 sous forme d'une poudre rouge :
- Point de Fusion : 52-53°C

| - Analyse élémentaire pour **C**_{**16**} **H**_{**33**} **N**_{**3**} **O**_{**4**} **Si**_{**3**} | | | | |
|---|---|---|---|---|
| théorie | **C** 46.23 | **H** 8.00 | **N** 10.11 | **Si** 20.27 |
| trouvé | **C** 46.40 | **H** 7.95 | **N** 10.16. | **Si** 20.16 |

### EXEMPLE 3

Dans un ballon tout équipé, on introduit 17,8 g (0,1 mole) du composé de formule : 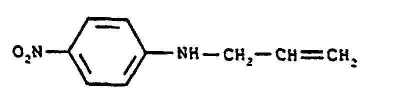 100 ml de toluène anhydre, et 100 µl de catalyseur au platine (complexe à 3-3,5% de Pt dans du cyclovinylméthylsiloxane, vendu par la société Hüls sous la marque Petrarch PC085). Le mélange est porté à 80°C sous azote, puis on ajoute goutte à goutte, 24,5 g (0,11 mole) d'heptaméthyltrisiloxane. Après quatre heures d'agitation à 80°C sous azote, on concentre le milieu, on évapore le solvant et le siloxane en excès.

L'huile obtenue est cristallisée dans un mélange Eau/Ethanol. On récupère ainsi 18,7 g du colorant siloxanique de formule (6) : 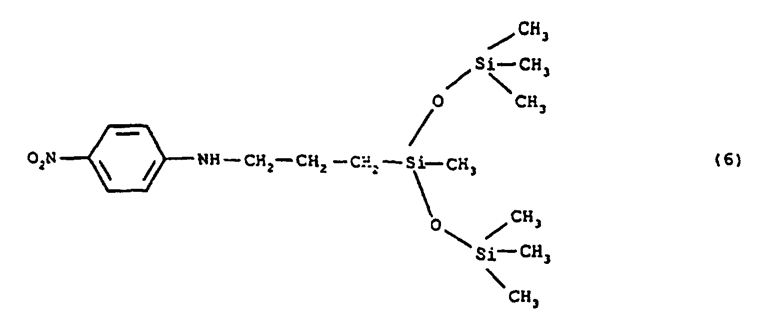 sous forme d'une poudre jaune :
- Point de Fusion : 39-40°C

| - Analyse élémentaire pour **C**₁₆ **H**₃₂ **N**₂ **O**₄ **Si**₃ | | | | |
|---|---|---|---|---|
| théorie | **C** 47.96 | **H** 8.05 | **N** 6.99 | **Si** 21.03 |
| trouvé | **C** 47.85 | **H** 8.31 | **N** 6.71 | **Si** 20.94 |

### EXEMPLE 4

Dans un ballon tout équipé, on introduit 4,46 g (0,02 mole) du composé de formule : 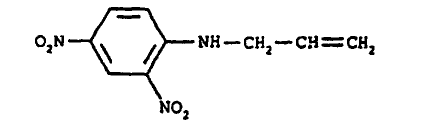 20 ml de toluène anhydre, et 20 µl de catalyseur au platine (complexe à 3-3,5% de Pt dans du cyclovinylméthylsiloxane, vendu par la société Hüls sous la marque Petrarch PC085). Le mélange est porté à 70°C sous azote, puis on ajoute goutte à goutte, 4,5 g (0,04 mole) d'heptaméthyltrisiloxane. Après trois heures d'agitation à 80°C sous azote, on concentre le milieu, on évapore le solvant et le siloxane en excès.
L'huile obtenue est chromatographiée sur silice (éluant : Heptane/CH₂Cl₂/90:10). On récupère ainsi 5,2 g du colorant siloxanique de formule (7) : 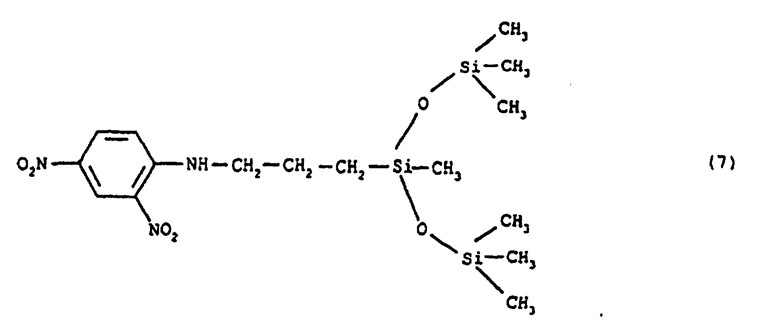 sous forme d'une poudre jaune-orangé :
- Point de Fusion : 56-57°C

| - Analyse élémentaire pour **C**_{**16**} **H**_{**31**} **N**_{**3**} **O**_{**6**} **Si**_{**3**} | | | | |
|---|---|---|---|---|
| théorie | **C** 43.12 | **H** 7.01 | **N** 9.43 | **Si** 18.90 |
| trouvé | **C** 43.14 | **H** 7.08 | **N** 9.41 | **Si** 19.05 |

### EXEMPLE 5

Dans un ballon tout équipé, on introduit 8,5 g (0,05 mole) du composé de formule : 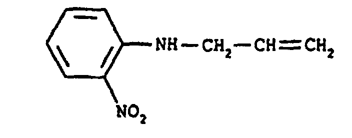 50 ml de toluène anhydre, et 100 µl de catalyseur au platine (complexe à 3-3,5% de Pt dans du cyclovinylméthylsiloxane, vendu par la société Hüls sous la marque Petrarch PC085). Le mélange est porté à 70°C sous azote, puis on ajoute goutte à goutte, 13,35 g (0,06 mole) d'heptaméthyltrisiloxane. Après trois heures d'agitation à 80°C sous azote, on concentre le milieu, on évapore le solvant et le siloxane en excès.
L'huile obtenue est chromatographiée sur silice (éluant : Heptane). On récupère ainsi 14,8 g du colorant siloxanique de formule (8) : 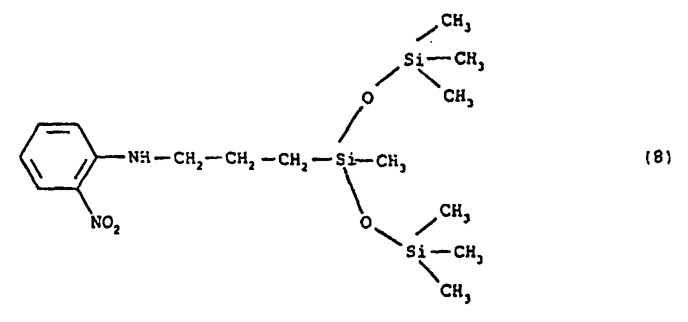 sous forme d'une huile orangée :

| - Analyse élémentaire pour **C**_{**16**} **H**_{**32**} **N**_{**2**} **O**_{**4**} **Si**_{**3**} | | | | |
|---|---|---|---|---|
| théorie | **C** 47.96 | **H** 8.05 | **N** 6.99 | **Si** 21.03 |
| trouvé | **C** 48.04 | **H** 7.96 | **N** 6.92 | **Si** 20.76 |

### EXEMPLE 6

Dans un ballon tout équipé, on introduit 1,675 g (0,0107 mole) du composé de formule : 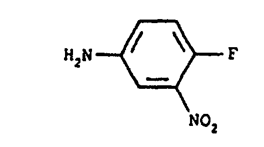 5 ml de dioxanne anhydre et 3 g (0,0107 mole) du composé de formule :

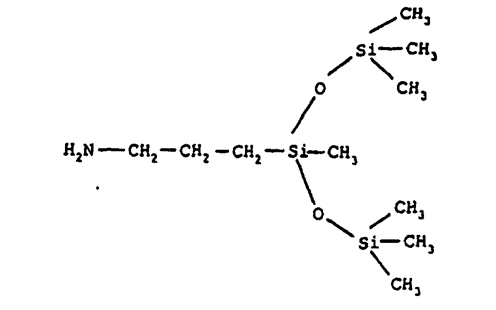

Le mélange est porté à 70°C sous azote pendant sept heures. On filtre à froid et le filtrat est concentré. Le résidu est cristallisé dans un mélange Eau/Ethanol/80:20. On récupère ainsi 0,2 g du colorant siloxanique de formule (9) 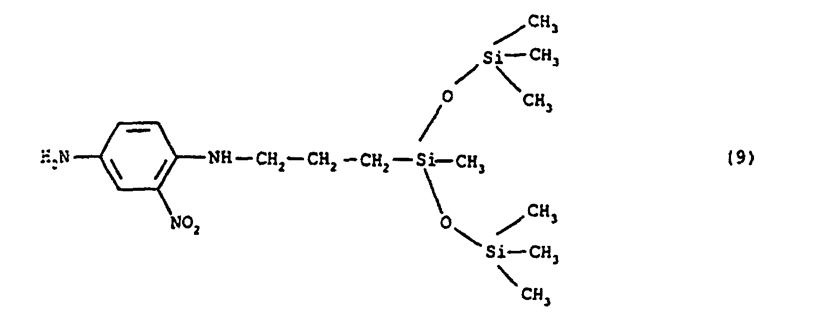 sous forme d'une poudre rouge noir :
- Point de Fusion : 81-82°C

| - Analyse élémentaire pour **C**_{**16**} **H**_{**33**} **N**_{**3**} **O**_{**4**} **Si**_{**3**} | | | | |
|---|---|---|---|---|
| théorie | **C** 46.23 | **H** 8.00 | **N** 10.11 | **Si** 20.27 |
| trouvé | **C** 46.26 | **H** 8.04 | **N** 9.92 | **Si** 20.50 |

### EXEMPLE 7 :

On a teint des mèches de cheveux gris naturels à 90% de blancs avec une composition de teinture contenant 5x10⁻² moles du colorant préparé à l'exemple 6, dans une quantité suffisante d'un mélange d'éthanol et d'eau (90/10 en poids) pour porter la composition à 100 g.
Après 30 minutes de traitement, on a rincé les cheveux à l'eau durant 5 minutes, puis on les a séchés.
Les mèches de cheveux ont été teintes en rouge-violacé.

### EXEMPLE 8 :

On a teint des mèches de cheveux gris naturels à 90% de blancs avec une composition de teinture contenant 5x10⁻² moles du colorant préparé à l'exemple 2, dans une quantité suffisante d'un mélange d'éthanol et d'eau (90/10 en poids) pour porter la composition à 100 g.
Après 30 minutes de traitement, on a rincé les cheveux à l'eau durant 5 minutes, puis on les a séchés.
Les mèches de cheveux ont été teintes en jaune-orangé.

## Revendications

1. Composés de formule 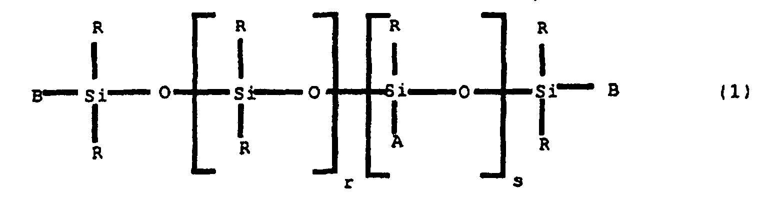 ou 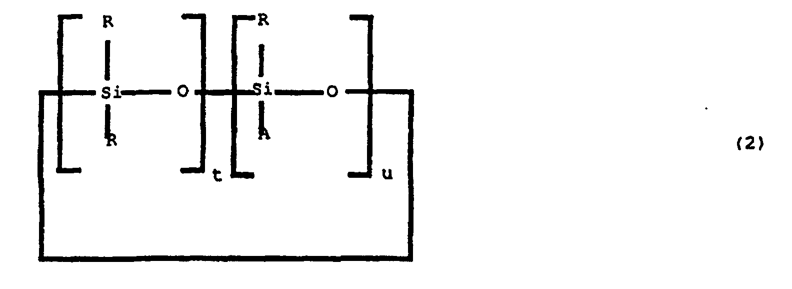 formule (1) ou (2) dans lesquelles :
- R, identiques ou différents, sont choisis parmi les radicaux alkyles linéaires ou ramifiés en C₁-C₁₀, phényle et trifluoro-3,3,3 propyle, au moins 80% en nombre des radicaux R étant méthyle,
- B, identiques ou différents, sont choisis parmi les radicaux R ci-dessus et le radical A défini ci-après,
- r est un nombre entier compris entre 0 et 50 inclusivement, et s est un nombre entier compris entre 0 et 20 inclusivement, avec la condition que si s est nul alors au moins l'un des deux symboles B désigne A,
- u est un nombre entier compris entre 1 et 6 inclusivement, et t est un nombre entier compris entre 0 et 10 inclusivement, étant entendu que t + u est égal ou supérieur à 3,
- et le symbole A désigne un radical monovalent lié directement à un atome de silicium, et qui répond à la formule (3) suivante : 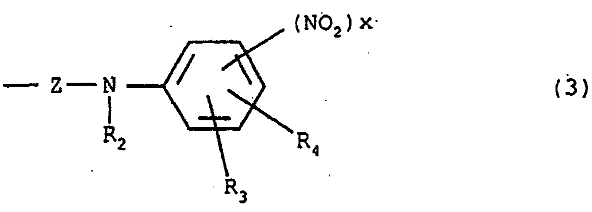 formule (3) dans laquelle :
- Z est le radical divalent : 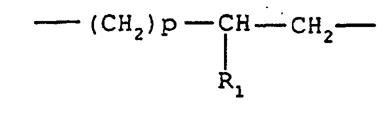
- x est 1 ou 2,
- p représente un nombre entier compris entre 0 et 10, inclusivement,
- R₁ représente l'hydrogène ou un radical alkyle en C₁-C₄,
- R₂ représente l'hydrogène ou un radical alkyle en C₁-C₄, ou le radical divalent Z défini ci-dessus,
- R₃ représente l'hydrogène ou un radical NR₅R₆ dans lequel R₅ et R₆ représentent l'hydrogène ou un radical alkyle en C₁-C₄, un radical mono- ou di-hydroxyalkyle en C₂-C₄ ou le radical divalent Z,
- R₄ représente l'hydrogène, un radical OH ou halogène, un radical alkyle en C₁-C₄, ou un radical alkoxy en C₁-C₄.

2. Composés selon la revendication 1 répondant à la formule (1) ou à la formule (2), **caractérisés par le fait que** les radicaux R sont des radicaux méthyle, éthyle, propyle, n-butyle, n-octyle ou éthyl-2 hexyle.

3. Composés selon la revendication 2, **caractérisés par le fait que** les radicaux R sont des radicaux méthyle.

4. Composés selon l'une quelconque des revendications précédentes, répondant à la formule (1), **caractérisés par le fait que** les radicaux B sont des radicaux méthyle, éthyle, propyle, n-butyle, n-octyle ou éthyl-2 hexyle.

5. Composés selon la revendication 4, **caractérisés par le fait que** les radicaux B sont radicaux méthyle.

6. Composés selon l'une quelconque des revendications précédentes, répondant à la formule (1), **caractérisés par le fait que** r est compris entre 0 et 3 inclusivement, et s est compris entre 0 et 3 inclusivement.

7. Composés selon l'une quelconque des revendications précédentes, répondant à la formule (1), **caractérisés par le fait que** r est nul et s est égal à 1.

8. Composés selon l'une quelconque des revendications 1 à 3, répondant à la formule (2), **caractérisés par le fait que** t + u est compris entre 3 et 5 inclusivement.

9. Composés selon l'une quelconque des revendications 1 à 3, répondant à la formule (2), **caractérisés par le fait que** t est égal à 2 et que u est égal à 1.

10. Composés selon l'une quelconque des revendications précédentes, **caractérisés par le fait que** R₁ est hydrogène ou méthyle.

11. Composés selon l'une quelconque des revendications précédentes, **caractérisés par le fait que** p est égal à 1.

12. Composés selon l'une quelconque des revendications précédentes, **caractérisés par le fait que**. R₂ et R₄ sont hydrogène.

13. Composés selon l'une quelconque des revendications précédentes, **caractérisés par le fait que**, R₃ est hydrogène, ou un radical NR₅R₆ dans lequel R₅ et R₆ représentent l'hydrogène ou un radical mono- ou di-hydroxyalkyle en C₂-C₄.

14. Composés selon l'une quelconque des revendications précédentes, **caractérisés par le fait qu'**ils répondent à l'une des formules suivantes :

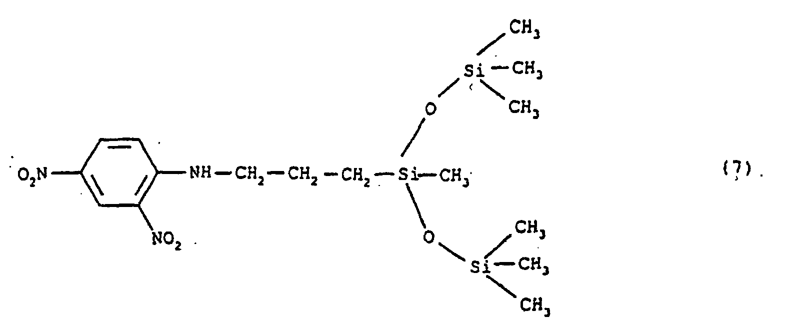

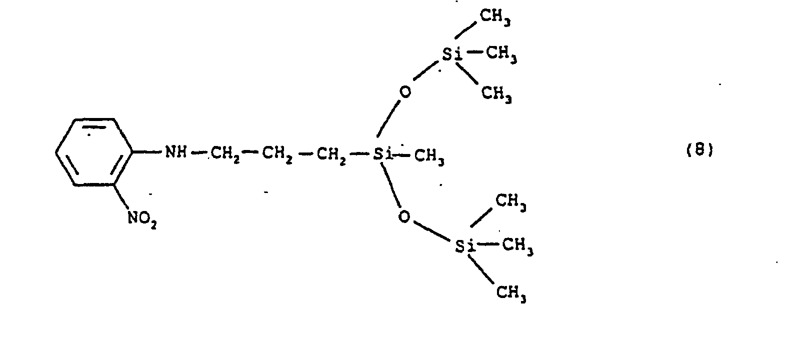

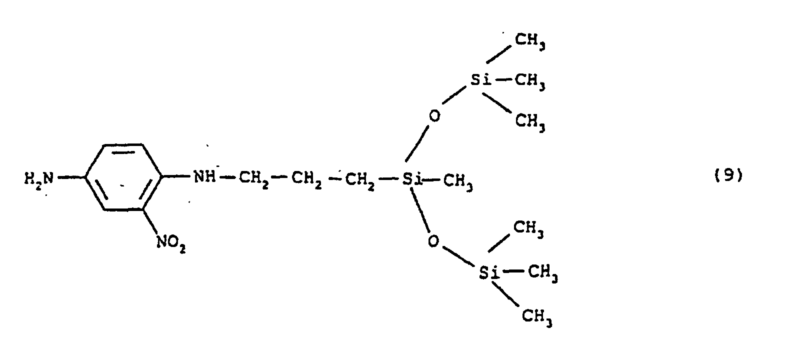

15. Utilisation des composés de formule (1) ou (2) tels que définis à l'une quelconque des revendications précédentes, à titre de colorants dans des, ou pour la préparation de, compositions cosmétiques.

16. Utilisation des composés de formule (1) ou (2) tels que définis à l'une quelconque des revendications précédentes, à titre de colorants dans des, ou pour la préparation de, compositions cosmétiques destinées à la teinture des fibres kératiniques.

17. Utilisation des composés de formule (1) ou (2) tels que définis à l'une quelconque des revendications 1 à 14, à titre de colorants dans des, ou pour la préparation de, compositions cosmétiques destinées au maquillage.

18. Utilisation des composés de formule(1) ou (2) tels que définis à l'une quelconque des revendications 1à 14, à titre de colorants directs dans des, ou pour la préparation de, compositions cosmétiques destinées à la teinture directe des cheveux.

19. Utilisation des composés de formule (1) ou (2) tels que définis à l'une quelconque des revendications 1 à 14, à titre de colorants directs dans des, ou pour la préparation de, compositions cosmétiques destinées à la teinture d'oxydation des cheveux.

20. Utilisation des composés de formule (1) ou (2) tels que définis à l'une quelconque des revendications 1 à 14 à titre de colorants dans des, ou pour la préparation de, compositions pharmaceutiques ou alimentaires.

21. Utilisation des composés de formule (1) ou (2) tels que définis à l'une quelconque des revendications 1 à 14, à titre de colorants dans des, ou pour la préparation de, compositions tinctoriales destinées à la teinture des fibres naturelles ou synthétiques, ou des matières plastiques ou minérales.

22. Composition cosmétique, **caractérisée par le fait qu'**elle comprend, dans un milieu cosmétiquement acceptable, une quantité efficace d'au moins un des composés définis à l'une quelconque des revendications 1 à 14.

23. Composition selon la revendication 22, **caractérisée par le fait que** le milieu cosmétiquement acceptable est constitué par de l'eau et/ou des solvants organiques tels que des alcools, des glycols, des éthers de glycols, et/ou des corps gras tels que des huiles et des cires.

24. Composition selon la revendication 22, **caractérisée par le fait que** la teneur en composé colorant est comprise entre 0,01 et 10% en poids par rapport au poids total de la composition.

25. Procédé de teinture des fibres kératiniques humaines et notamment des cheveux, par coloration directe, **caractérisé par le fait qu'**on applique une composition tinctoriale renfermant au moins un composé de formule (1) ou (2) tel que défini à l'une quelconque des revendications 1 à 14, sur les fibres kératiniques, sèches ou humides, et qu'après avoir éventuellement laissé agir la composition sur les fibres pendant un temps de pose allant de 3 à 60 minutes, on sèche ces fibres, après un rinçage éventuel.

## Patentansprüche

1. Verbindungen der Formel 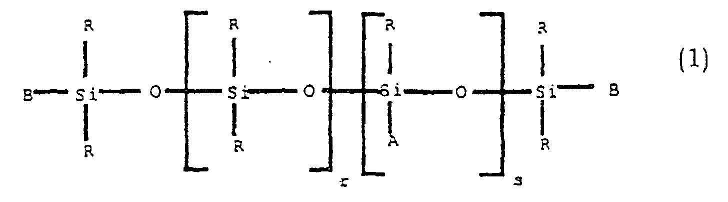 oder 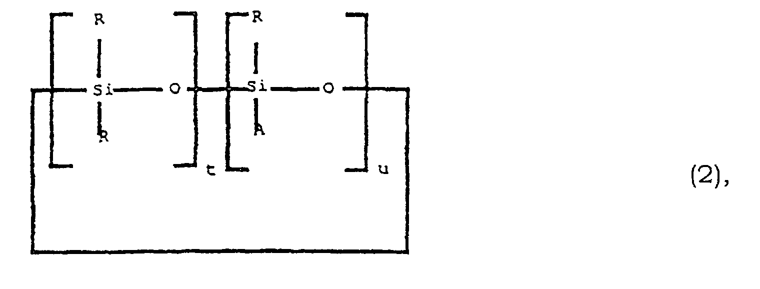 in denen bedeuten:
- R, gleich oder verschieden, Reste, die unter geradkettigem oder verzweigtem C₁-C₁₀-Alkyl, Phenyl, 3,3,3-Trifluorpropyl ausgewählt sind, wobei mindestens 80 % der Reste R, bezogen auf die Zahl der Reste, Methyl bedeuten,
- B, gleich oder verschieden, Reste, die unter den obigen Resten R und den weiter unten definierten Rest A ausgewählt sind,
- r eine ganze Zahl im Bereich von 0 bis 50 und s eine ganze Zahl im Bereich von 0 bis 20 mit der Maßgabe, daß mindestens eines der beiden Symbole B einen Rest A bedeutet, wenn s gleich Null ist,
- u eine ganze Zahl im Bereich von 1 bis 6 und t eine ganze Zahl im Bereich von 0 bis 10, wobei die Summe t + u größer als oder gleich 3 ist, und
- das Symbol A einen einwertigen Rest, der unmittelbar mit einem Siliciumatom verbunden ist und der folgenden Formel (3) 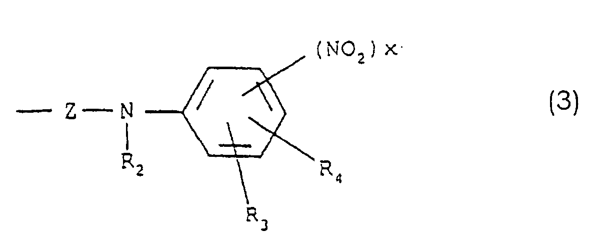 entspricht, in der bedeuten:
- Z den zweiwertigen Rest 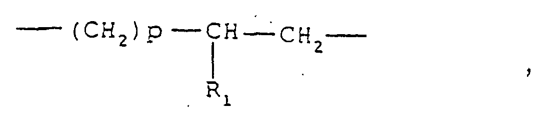
- x die Zahl 1 oder 2,
- p eine ganze Zahl im Bereich von 0 bis 10,
- R₁ Wasserstoff oder C₁-C₄-Alkyl,
- R₂ Wasserstoff, C₁-C₄-Alkyl oder den oben definierten zweiwertigen Rest Z,
- R₃ Wasserstoff oder einen Rest NR₅R₆, in dem R₅ und R₆ Wasserstoff oder C₁-C₄-Alkyl, einen Monohydroxy- oder Dihydroxy-C₂-C₄-alkyl oder den zweiwertigen Rest Z bedeuten,
- R₄ Wasserstoff, eine Hydroxygruppe oder ein Halogen, C₁-C₄-Alkyl oder C₁-C₄-Alkoxy.

2. Verbindungen nach Anspruch 1 der Formel (1) oder der Formel (2), **dadurch gekennzeichnet, daß** es sich bei den Resten R um Methyl-, Ethyl-, Propyl-,. n-Butyl-, n-Octyl- oder 2-Ethylhexylreste handelt.

3. Verbindungen nach Anspruch 2, **dadurch gekennzeichnet, daß** die Reste R Methylreste sind.

4. Verbindungen nach einem der vorhergehenden Ansprüche, die der Formel (1) entsprechen, **dadurch gekennzeichnet, daß** es sich bei den Resten B um Methyl-, Ethyl-, Propyl-, n-Butyl-, n-Octyl- oder 2-Ethylhexylreste handelt.

5. Verbindungen nach Anspruch 4, **dadurch gekennzeichnet, daß** die Reste B Methylreste sind.

6. Verbindungen nach einem der vorhergehenden Ansprüche, die der Formel (1) entsprechen, **dadurch gekennzeichnet, daß** r im Bereich von 0 bis 3 liegt und daß s im Bereich von 0 bis 3 liegt.

7. Verbindungen nach einem der vorhergehenden Ansprüche, die der Formel (1) entsprechen, **dadurch gekennzeichnet, daß** r Null ist und s gleich 1 ist.

8. Verbindungen nach einem der Ansprüche 1 bis 3, die der Formel (2) entsprechen, **dadurch gekennzeichnet, daß** die Summe t + u im Bereich von 3 bis 5 liegt.

9. Verbindungen nach einem der Ansprüche 1 bis 3, die der Formel (2) entsprechen, **dadurch gekennzeichnet, daß** t gleich 2 ist und daß u gleich 1 ist.

10. Verbindungen nach einem der vorhergehenden Ansprüche, **dadurch gekennzeichnet, daß** R₁ Wasserstoff oder Methyl bedeutet.

11. Verbindungen nach einem der vorhergehenden Ansprüche, **dadurch gekennzeichnet, daß** p gleich 1 ist.

12. Verbindungen nach einem der vorhergehenden Ansprüche, **dadurch gekennzeichnet, daß** R₂ und R₄ Wasserstoff bedeuten.

13. Verbindungen nach einem der vorhergehenden Ansprüche, **dadurch gekennzeichnet, daß** R₃ Wasserstoff oder einen Rest NR₅R₆ bedeutet, worin R₅ und R₆ Wasserstoff oder Monohydroxy- oder Dihydroxy-C₂-C₄-Alkyl darstellen.

14. Verbindungen nach einem der vorhergehenden Ansprüche, **dadurch gekennzeichnet, daß** sie einer der folgenden Formeln entsprechen

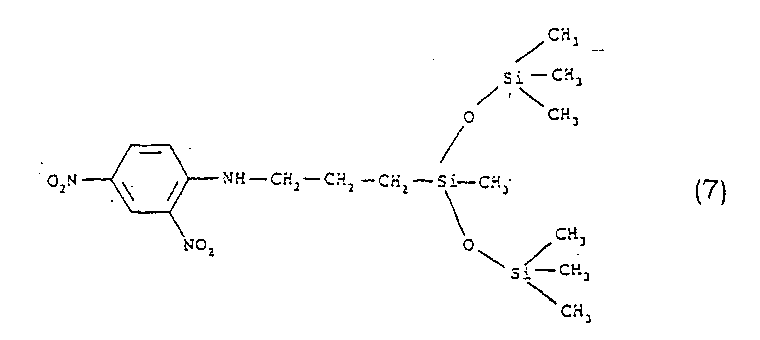

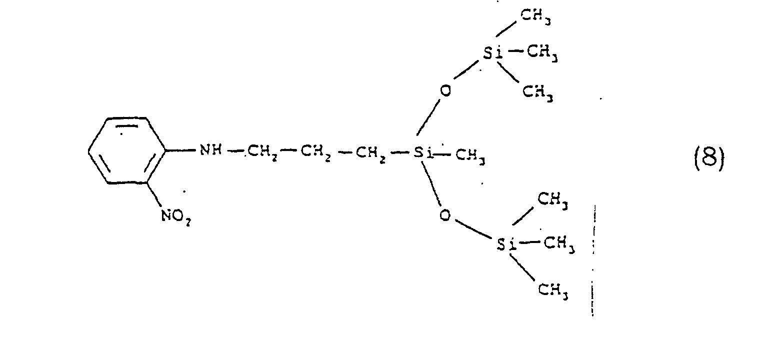

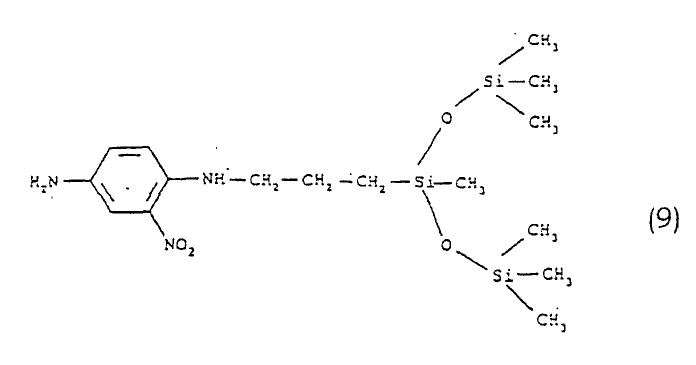

15. Verwendung der Verbindungen der Formel (1) oder (2), die wie in einem der vorhergehenden Ansprüche definiert sind, als Farbstoffe in oder für die Herstellung von kosmetischen Zusammensetzungen.

16. Verwendung der Verbindungen der Formel (1) oder (2), die wie in einem der Ansprüche 1 bis 14 definiert sind, als Farbstoffe in oder für die Herstellung von kosmetischen Zusammensetzungen, die für die Färbung von Keratinfasern vorgesehen sind.

17. Verwendung der Verbindungen der Formel (1) oder (2), die wie in einem der Ansprüche 1 bis 14 definiert sind, als Farbstoffe in oder für die Herstellung von kosmetischen Zusammensetzungen, die zum Schminken vorgesehen sind.

18. Verwendung der Verbindungen der Formel (1) oder (2), die wie in einem der Ansprüche 1 bis 14 definiert sind, als direktziehende Farbstoffe in oder für die Herstellung von kosmetischen Zusammensetzungen, die für die direktziehende Färbung der Haare vorgesehen sind.

19. Verwendung der Verbindungen der Formel (1) oder (2), die wie in einem der Ansprüche 1 bis 14 definiert sind, als Farbstoffe in oder für die Herstellung von kosmetischen Zusammensetzungen, die für die oxidative Färbung der Haare vorgesehen sind.

20. Verwendung der Verbindungen der Formel (1) oder (2), die wie in einem der Ansprüche 1 bis 14 definiert sind, als Farbstoffe in oder für die Herstellung von pharmazeutischen Zusammensetzungen oder Lebensmittelzusammensetzungen.

21. Verwendung der Verbindungen der Formel(1) oder (2), die wie in einem der Ansprüche 1 bis 14 definiert sind, als Farbstoffe in oder für die Herstellung von Färbemittelzusammensetzungen, die für die Färbung natürlicher oder synthetischer Fasern oder von Kunststoffmaterialien oder anorganischen Materialien vorgesehen sind.

22. Kosmetische Zusammensetzung, **dadurch gekennzeichnet, daß** sie in einem kosmetisch akzeptablen Medium eine wirksame Menge mindestens einer der in einem der Ansprüche 1 bis 14 definierten Verbindungen enthält.

23. Zusammensetzung nach Anspruch 22, **dadurch gekennzeichnet, daß** das kosmetisch akzeptable Medium aus Wasser und/oder organischen Lösemitteln, wie Alkoholen, Glykolen, Glykolethern und/oder Fettsubstanzen, wie Ölen und Wachsen, besteht.

24. Zusammensetzung nach Anspruch 22, **dadurch gekennzeichnet, daß** der Gehalt an färbender Verbindung im Bereich von 0,01 bis 10 Gew.-%, bezogen auf das Gesamtgewicht der Zusammensetzung, liegt.

25. Verfahren zum Färben menschlicher Keratinfasern und insbesondere der Haare durch direktziehende Färbung, **dadurch gekennzeichnet, daß** eine Färbemittelzusammensetzung, die mindestens eine Verbindung der Formel (1) oder (2), die wie in einem der Ansprüche 1 bis 14 definiert ist, enthält, auf die trockenen oder feuchten Keratinfasern aufgetragen wird und daß die Fasern, nachdem man die Zusammensetzung gegebenenfalls während einer Einwirkzeit von 3 bis 60 min auf die Fasern hat einwirken lassen, nach einem gegebenenfalls durchgeführten Ausspülen trocknet.

## Claims

1. Compounds of formula: 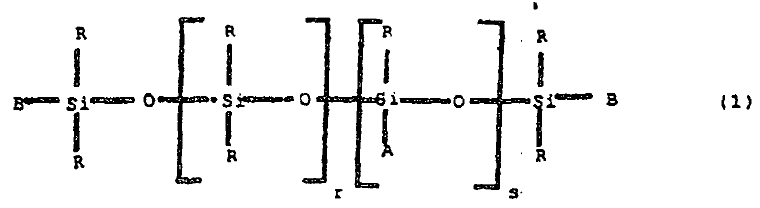 or 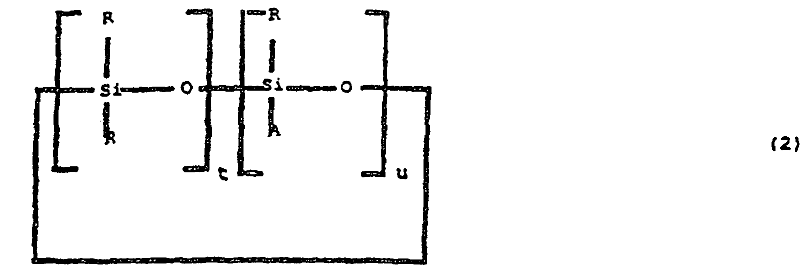 in which formula (1) or (2):
- R, which may be identical or different, are chosen from linear or branched C₁-C₁₀ alkyl, phenyl and 3,3,3-trifluoropropyl radicals, at least 80% in numerical terms of the radicals R being methyl,
- B, which may be identical or different, are chosen from the radicals R above and the radical A defined below,
- r is an integer between 0 and 50 inclusive and s is an integer between 0 and 20 inclusive, with the condition that if s is zero then at least one of the two symbols B denotes A,
- u is an integer between 1 and 6 inclusive, and t is an integer between 0 and 10 inclusive, it being understood that t + u is greater than or equal to 3,
- and the symbol A denotes a monovalent radical directly linked to a silicon atom, and which corresponds to formula (3) below: 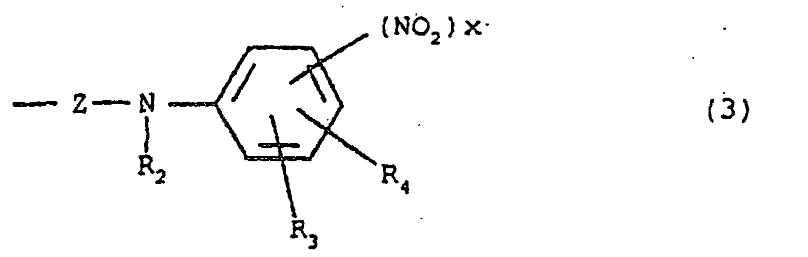 in which formula (3):
- Z is a divalent radical: 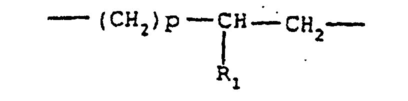
- x is 1 or 2,
- p represents an integer between 0 and 10 inclusive,
- R₁ represents hydrogen or a C₁-C₄ alkyl radical,
- R₂ represents hydrogen or a C₁-C₄ alkyl radical, or the divalent radical Z defined above,
- R₃ represents hydrogen or a radical NR₅R₆ in which R₅ and R₆ represent hydrogen or a C₁-C₄ alkyl radical, a C₂-C₄ monohydroxyalkyl or dihydroxyalkyl radical or a divalent radical Z,
- R₄ represents hydrogen, an OH or halogen radical, a C₁-C₄ alkyl radical or a C₁-C₄ alkoxy radical.

2. Compounds according to Claim 1 corresponding to formula (1) or to formula (2), **characterized in that** the radicals R are methyl, ethyl, propyl, n-butyl, n-octyl or 2-ethylhexyl radicals.

3. Compounds according to Claim 2, **characterized in that** the radicals R are methyl radicals.

4. Compounds according to any one of the preceding claims, corresponding to formula (1), **characterized in that** the radicals B are methyl, ethyl, propyl, n-butyl, n-octyl or 2-ethylhexyl radicals.

5. Compounds according to Claim 4, **characterized in that** the radicals B are methyl radicals.

6. Compounds according to any one of the preceding claims, corresponding to formula (1), **characterized in that** r is between 0 and 3 inclusive and s is between 0 and 3 inclusive.

7. Compounds according to any one of the preceding claims, corresponding to formula (1), **characterized in that** r is zero and s is equal to 1.

8. Compounds according to any one of Claims 1 to 3, corresponding to formula (2), **characterized in that** t + u is between 3 and 5 inclusive.

9. Compounds according to any one of Claims 1 to 3, corresponding to formula (2), **characterized in that** t is equal to 2 and u is equal to 1.

10. Compounds according to any one of the preceding claims, **characterized in that** R₁ is hydrogen or methyl.

11. Compounds according to any one of the preceding claims, **characterized in that** p is equal to 1.

12. Compounds according to any one of the preceding claims, **characterized in that** R₂ and R₄ are hydrogen.

13. Compounds according to any one of the preceding claims, **characterized in that** R₃ is hydrogen or a radical NR₅R₆ in which R₅ and R₆ represent hydrogen or a C₂-C₄ monohydroxyalkyl or dihydroxyalkyl radical.

14. Compounds according to any one of the preceding claims, **characterized in that** they correspond to one of the following formulae:

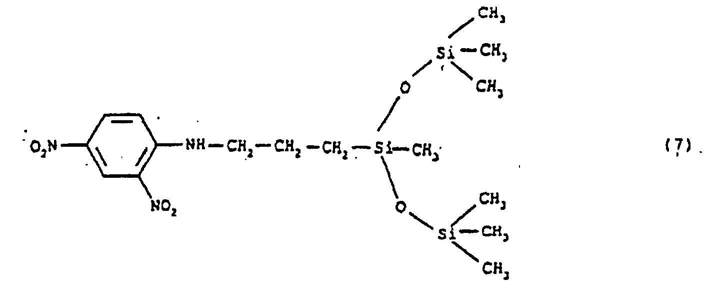

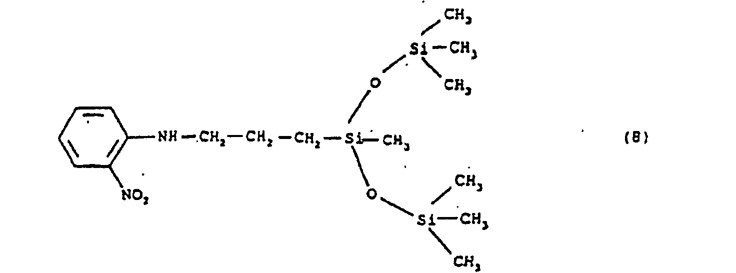

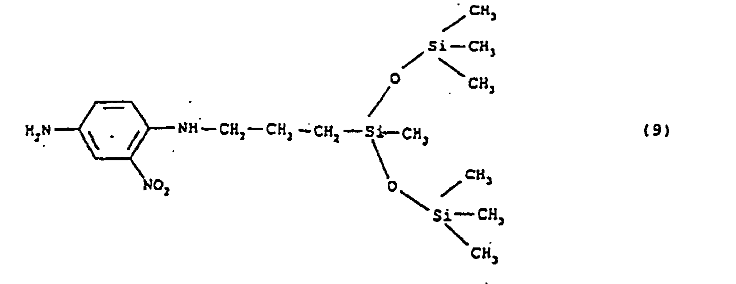

15. Use of the compounds of formula (1) or (2) as defined in any one of the preceding claims, as colorants in or for the preparation of cosmetic compositions.

16. Use of the compounds of formula (1) or (2) as defined in any one of the preceding claims, as colorants in or for the preparation of cosmetic compositions for dyeing keratin fibres.

17. Use of the compounds of formula (1) or (2) as defined in any one of Claims 1 to 14, as colorants in or for the preparation of cosmetic compositions for make-up.

18. Use of the compounds of formula (1) or (2) as defined in any one of Claims 1 to 14, as direct dyes in or for the preparation of cosmetic compositions for the direct dyeing of the hair.

19. Use of the compounds of formula (1) or (2) as defined in any one of Claims 1 to 14, as direct dyes in or for the preparation of cosmetic compositions for the oxidation dyeing of the hair.

20. Use of the compounds of formula (1) or (2) as defined in any one of Claims 1 to 14, as colorants in or for the preparation of pharmaceutical or food compositions.

21. Use of the compounds of formula (1) or (2) as defined in any one of Claims 1 to 14, as colorants in or for the preparation of dye compositions for dyeing natural or synthetic fibres, or plastics or minerals.

22. Cosmetic composition, **characterized in that** it comprises, in a cosmetically acceptable medium, an effective amount of at least one of the compounds defined in any one of Claims 1 to 14.

23. Composition according to Claim 22, **characterized in that** the cosmetically acceptable medium consists of water and/or organic solvents such as alcohols, glycols, glycol ethers and/or fatty substances such as oils and waxes.

24. Composition according to Claim 22, **characterized in that** the content of colorant compound is between 0.01% and 10% by weight relative to the total weight of the composition.

25. Process for dyeing human keratin fibres, and in particular the hair, by direct dyeing, **characterized in that** a dye composition containing at least one compound of formula (1) or (2) as defined in any one of Claims 1 to 14 is applied to wet or dry keratin fibres and, after the composition has optionally been left to act on the fibres for an exposure time ranging from 3 to 60 minutes, these fibres are dried, after an optional rinsing.
